# EUROPÄISCHE PATENTSCHRIFT

(11) **EP 2 975 111 B1**
(45) Veröffentlichungstag und Bekanntmachung des Hinweises auf die Patenterteilung: **16.05.2018**
(21) Anmeldenummer: 14002424.1
(22) Anmeldetag: 14.07.2014
(51) Int. Cl.: C12M 1/00, C12M 3/00, C12M 3/06

(54) **Mischvorrichtung zum Mischen eines Inhalts eines Behälters**
Mixing device for mixing a content of a container
Dispositif de mélange d'un contenu d'un récipient

(43) Veröffentlichungstag der Anmeldung: 20.01.2016
(73) Patentinhaber: Sartorius Stedim Biotech GmbH, 37079 Göttingen (DE); Sartorius Stedim Switzerland AG, 8317 Tagelswangen (CH)
(72) Erfinder: Röll, Marcel, 8124 Maur, ZH (CH); Hoepli, Daniel, 8362 Balterwil, TG (CH)
(74) Vertreter: Müller-Boré & Partner Patentanwälte PartG mbB

(56) Entgegenhaltungen:
- EP-B1- 1 173 542
- WO-A1-00/66706
- WO-A1-2012/115581
- DE-A1-102005 007 512
- US-A1- 2013 244 322

## Beschreibung

Die vorliegende Erfindung betrifft eine Mischvorrichtung zum Mischen eines Inhalts eines Behälters.

In vielen industriellen Bereichen werden Behältnisse zur Durchmischung oder Umwälzung von Stoffen verwendet. Bei der Herstellung von Kulturmedien für Microorganismen oder bei kontrollierten biotechnologischen Prozessen wie der Zellkultivierung kommt der Durchmischung bzw. dem Umwälzen des Behälterinhaltes eine besondere Bedeutung zu. Aufgrund der unterschiedlichen Ausgangskonzentrationen der Stoffe sowie der Stoffwechselaktivität der Mikroorganismen während der Kultivierung kommt es zu lokalen Konzentrationsänderungen verschiedenster chemischer Komponenten im Medienansatz oder von Nährstoffen, Sauerstoff und den entstehenden Metaboliten während der Kultivierung. Um im gesamten Behältnis gleiche oder zumindest kontrollierte Konzentrationsbedingungen zu gewährleisten, ist es erforderlich, die Flüssigkeit oder Suspension im Behälter während des gesamten Prozesses durchzumischen bzw. umzuwälzen.

Der Einsatz von flexiblen Einwegbioreaktoren aus Folien nimmt dabei insbesondere im Hinblick auf die ständig steigenden Anforderungen an die Sterilität der Prozesse in der Bioverfahrenstechnik gegenüber starren Behältern aus Glas oder Edelstahl stetig an Bedeutung zu. Neben der guten Sterilisierbarkeit bieten die Folienbeutel weitere Vorteile wie die kostengünstige Herstellung, die einfache und platzsparende Lagerung, die weitestgehende Sicherheit gegenüber Kontamination und den Entfall einer aufwendigen Reinigung nach dem Gebrauch.

Die Durchmischung des Inhaltes der flexiblen Einwegbioreaktoren erfolgt sowohl mechanisch unter Zuhilfenahme von Rührorganen als auch über die Bewegung des Behälters mit der darin enthaltenen Flüssigkeit selbst. Insbesondere bei der Verwendung von flexiblen Behältern zum Einmalgebrauch wird der Einsatz von rührerlosen Mischern als vorteilhaft angesehen, da keine Durchgänge durch die Behälterwand zum Antreiben von Rührelementen notwendig sind. Je nach Art der Bewegung sind derartige Mischer als Rocker, Plattformschüttler, Taumelmischer, Kreisschüttler, Vibrationsschüttler, Horizontalschüttler, Orbital-Shaker etc., bekannt.

Flexible Behälter können je nach Einsatzgebiet verschiedene Größen aufweisen, wobei größere Behälter Volumina von mehreren Litern aufweisen können. Die meistens vorher befüllten Behälter werden üblicherweise auf plattenförmigen oder wannenförmigen beweglichen Trägern angeordnet, wobei sie z.B. mittels Klemmen oder Haken oder sonstigen lösbaren Befestigungsmitteln auf den Trägern fixiert werden. Die Träger werden angetrieben und erzeugen die Mischbewegung des Inhalts des Behälters. Der Träger führt hierbei beispielsweise eine Wippbewegung um eine horizontale Lage aus.

Um den Inhalt der Beutel wieder zu entnehmen, lassen sich die Träger mit den darauf fixierten Beuteln in eine nahezu vertikale Position bewegen. Auch ein Befüllen der Behälter in der vertikalen Position ist ggf. möglich.

WO 2012/115581 A1 offenbart einen Bioreaktor mit einer Mischvorrichtung, bei welcher ein Behälter-Träger eine Wippbewegung um eine horizontale Wippachse zum Mischen eines Inhalts eines Behälters ausführt. An einem vorderen Ende des Behälter-Trägers ist eine Aufstell-Achse angeordnet, um welche der Behälter-Träger zum Aufstellen des Behälter-Trägers geschwenkt werden kann, wobei die Aufstell-Achse parallel zur Wippachse verläuft. Um die Entnahme von Produkten aus dem Behälter zu vereinfachen, kann der Behälter-Träger im aufgestellten Zustand um eine zweite Kipp-Achse, welche senkrecht zur Aufstell-Achse verläuft, zu einer Ecke des Behälters hin geneigt werden. Durch das Vorsehen einer Aufstell-Achse einerseits und einer dazu senkrechten Kipp-Achse andererseits ist die Konstruktion der Mischvorrichtung jedoch aufwendig und in der Handhabung unvorteilhaft.

US 2013/0244322 A1 offenbart einen Bioreaktor mit einer Mischvorrichtung, bei welcher ein Behälter-Träger eine Wippbewegung um eine horizontale Wippachse zum Mischen eines Inhalts eines Behälters ausführt. An einem vorderen Ende des Behälter-Trägers ist eine horizontale Aufstell-Achse angeordnet, um welche der Behälter-Träger zum Aufstellen des Behälter-Trägers geschwenkt werden kann, wobei die Aufstell-Achse parallel zur Wippachse verläuft.

Es ist Aufgabe der vorliegenden Erfindung, eine verbesserte Mischvorrichtung bereitzustellen, welche auf einfache Weise einen Behälter in eine vorteilhafte Befüll- bzw. Entnahmestellung verlagern kann.

Diese Aufgabe wird durch den Gegenstand des unabhängigen Anspruchs gelöst. Bevorzugte Ausführungsformen ergeben sich aus den abhängigen Ansprüchen.

Gemäß der vorliegenden Erfindung wird eine Mischvorrichtung zum Mischen eines Inhalts eines Behälters bereitgestellt, aufweisend: einen Behälter-Träger zum Tragen des Behälters; und eine Bewegungsvorrichtung, welche zum Bewegen des Behälter-Trägers mit dem Behälter-Träger verbunden ist, um den Inhalt des Behälters in einer Mischstellung mischen zu können; wobei der Behälter-Träger eine Positioniervorrichtung aufweist, welche ausgelegt ist, den Behälter in einer vorbestimmten bzw. vorbestimmbaren Lage an dem Behälter-Träger zu positionieren, so dass eine Entnahmeseite des Behälters im Wesentlichen entlang einer vorbestimmten Linie des Behälter-Trägers angeordnet ist, wobei der Behälter-Träger um eine Entnahme-Schwenkachse schwenkbar relativ zu der Bewegungsvorrichtung gelagert ist, um den daran positionierbaren Behälter in eine Entnahmestellung verlagern zu können, wobei die Entnahme-Schwenkachse derart in einem Schwenkachsenwinkel von über 0° bis 45° zur vorbestimmten Linie des Behälter-Trägers angeordnet ist, dass der daran positionierbare Behälter von der Mischstellung mittels einer Schwenkbewegung des Behälter-Trägers um die Entnahme-Schwenkachse in die Entnahmestellung verlagerbar ist, in der die Entnahmeseite an der unteren Seite des Behälters befindlich sowie zu einem Entnahmebereich des Behälters hin geneigt ist.

Bei dem Behälter kann es sich insbesondere um einen Bioreaktorbehälter handeln, wobei dieser aus einem Folienmaterial hergestellt sein kann. Ein derartiger Behälter kann als Beutel mit zumindest an zwei gegenüberliegenden Seiten verschweißten Rändern ausgeführt sein. Der Behälter kann eine vorbestimmte Auflagefläche aufweisen, auf welcher der Behälter stabil auf einer horizontalen Fläche abgelegt werden kann. Die Auflagefläche kann insbesondere eine seiner größten Oberflächen bzw. Seitenflächen sein. Die Auflagefläche und/oder die der Auflagefläche gegenüberliegende Fläche können Durchgänge und Anschlüsse für Zufuhr- und/oder Abfuhrleitungen und/oder Sensoren aufweisen. Sofern sich die Zufuhr- und/oder Abfuhrleitungen an der Auflagefläche befinden, verfügt auch der Behälter-Träger über entsprechende Durchgänge für die angeschlossenen Leitungen. Der Behälter kann mit seiner Auflagefläche auf dem Behälter-Träger angeordnet werden. Der Behälter kann in der Draufsicht eine im Wesentlichen viereckige Form mit vier Seitenkanten aufweisen. Insbesondere kann der Behälter in der Draufsicht symmetrisch ausgebildet sein, was für eine gleichmäßige Bewegung des Inhalts des Behälters während des Mischens vorteilhaft ist. Zumindest weist der Behälter eine Entnahmeseite auf. Die Entnahmeseite kann eine im Wesentlichen gerade Seitenkante des Behälters sein, wobei eine Seitenkante des Behälterinnenraums gemeint ist. Die Entnahmeseite bezeichnet die Seite des Behälters, welche zum Sammeln und zur Entnahme des Inhalts des Behälters und insbesondere der Produkte, die sich im Behälter gebildet haben, vorgesehen ist. Der Entnahmebereich des Behälters kann in einer der zwei Ecken der Entnahmeseite angeordnet sein. Am Entnahmebereich kann ein Auslass angeordnet sein, aus welchem der Inhalt des Behälters ausgelassen werden können.

Vorzugsweise wird der an dem Behälter-Träger positionierte Behälter zusammen mit dem Behälter-Träger zur Entnahme in die Entnahmestellung verlagert. Ein Befüllen des Behälters ist jedoch ebenfalls in dieser Stellung über die am Behälter angeordneten Zufuhrleitungen möglich.

Der Inhalt des Behälters kann eine Flüssigkeit oder eine Suspension sein. Der Behälter kann ferner ein Gas bzw. Gase enthalten. Der Inhalt des Behälters wird mittels der Mischvorrichtung gemischt bzw. umgewälzt, wodurch insbesondere bei der Zellkultivierung eine gute Sauerstoffdurchmischung gewährleistet werden kann.

Der Behälter-Träger ist derart ausgebildet, dass der Behälter darauf angeordnet werden kann. Der Behälter-Träger kann derart ausgebildet sein, dass der Behälter mit seiner dem Behälter-Träger zugewandten größten Fläche bzw. seiner Auflagefläche im Wesentlichen vollständig an der oberen Fläche des Behälter-Trägers anliegt. Die obere Fläche des Behälter-Trägers kann als Aufnahmefläche bezeichnet werden. Der Behälter-Träger kann beispielsweise platten- oder wannenförmig ausgebildet sein und in der Draufsicht im Wesentlichen viereckig ausgeführt sein. Die wannenförmigen Behälter-Träger dienen im Wesentlichen dazu, bei Leckagen der Behälter das austretende Fluid aus den Behältern aufzunehmen. Es sind jedoch auch wannenförmige Behälter-Träger mit einer oder mehreren offenen Ecken bzw. Durchbrüchen möglich, über die in der Aufstellposition die Abfuhrleitungen geführt werden um so eine Entnahme aus dem Behälter zu erleichtern. Die Größe des Behälter-Trägers ist nicht beschränkt und kann in Abhängigkeit von der Behältergröße gewählt werden. Vorzugsweise entspricht die Form und Größe des Behälter-Trägers in der Draufsicht im Wesentlichen der Form und Größe des Behälters in der Draufsicht.

Die Bewegungsvorrichtung ist ausgelegt, den Behälter-Träger zum Ausführen der Mischbewegung zu bewegen. Hierzu ist die Bewegungsvorrichtung, vorzugsweise über ein bewegliches Kopplungselement, mit dem Behälter-Träger verbunden. Beim Bewegen des Behälters zum Mischen des Inhalts des Behälters ist der Behälter-Träger in einer Mischlage, und der darauf positionierte Behälter ist in einer Mischstellung. In der Mischlage ist der Behälter-Träger, bzw. die Aufnahmefläche davon, im Wesentlichen horizontal ausgerichtet. Genauer gesagt führt der Behälter-Träger in der Mischlage zum Mischen eine Bewegung um oder in der horizontalen Ebene aus. Je nach Art der Bewegung kann die Mischvorrichtung als Rocker, Plattformschüttler, Taumelmischer, Kreisschüttler, Vibrationsschüttler, Horizontalschüttler, Orbital-Shaker etc., bezeichnet werden. Bei einem Rocker führt der Behälter-Träger eine Kipp- oder Wipp-Bewegung um eine im Wesentlichen horizontalen Achse aus, wobei der Behälter-Träger zunächst in einem Bereich von etwa 0-15°, vorzugsweise in einem Bereich von etwa 0-10°, zur horizontalen Ebene geneigt wird, und anschließend in umgekehrter Richtung in einem Bereich von etwa 0-15°, vorzugsweise in einem Bereich von etwa 0-10°, zur horizontalen Ebene geneigt wird. Diese Bewegung des Behälter-Trägers wird auf den Behälter übertragen, so dass eine Wellenbewegung des Inhalts des Behälters erzeugt werden kann. Die Mischbewegung des Behälter-Trägers muss jedoch nicht zwingend ein Neigen zur horizontalen Ebene beinhalten, und der Behälter-Träger kann stattdessen auch in der horizontalen Ebene linear oder kreisförmig hin und her bewegt werden und/oder horizontal ausgerichtet in vertikaler Richtung hin und her bewegt bzw. geschüttelt werden. Mischformen der Bewegungsarten sind ebenfalls möglich. Die Mischlage ist durch die Bewegung des Behälter-Trägers also veränderbar, liegt aber im Wesentlichen in der horizontalen Ebene bzw. weicht nur geringfügig von dieser ab. Vorzugsweise weist der Behälter-Träger eine vorbestimmte bzw. vorbestimmbare Ruhelage auf, welche eingenommen wird, wenn der Behälter-Träger in der Mischlage keine Mischbewegung ausführt. In der Ruhelage befindet sich der auf dem Behälter-Träger angeordnete Behälter in einer Ruhestellung. In der Ruhelage bzw. Ruhestellung ist der Behälter-Träger bzw. der Behälter vorzugsweise etwa horizontal ausgerichtet. Entsprechendes gilt für die Mischstellung des auf dem Behälter-Träger angeordneten Behälters.

Zum Positionieren bzw. Fixieren des Behälters an dem Behälter-Träger weist der Behälter-Träger eine Positioniervorrichtung auf, welche jeweils an einer oder an mehreren Seiten des Behälter-Trägers vorgesehen sein kann. Die Positioniervorrichtung kann beispielsweise Klemmen, Klemmschienen und/oder Befestigungshaken aufweisen. Insbesondere kann die Positioniervorrichtung ein lösbares Positionieren bzw. Fixieren des Behälters an dem Behälter-Träger ermöglichen. Die Positioniervorrichtung ist ausgelegt, den Behälter in einer vorbestimmten bzw. vorbestimmbaren Lage an bzw. auf dem Behälter-Träger zu positionieren, so dass die Entnahmeseite des Behälters im Wesentlichen entlang einer vorbestimmten gedachten Linie des Behälter-Trägers angeordnet ist. Die vorbestimmte Linie ist eine gedachte Linie und muss nicht auf dem Behälter-Träger sichtbar sein. Entlang dieser gedachten Linie des Behälter-Trägers erstreckt sich die Entnahmeseite des Behälters, insbesondere die im Wesentlichen gerade Seitenkante des Behälterinnenraums, wenn der Behälter mittels der Positioniervorrichtung an dem Behälter-Träger positioniert ist.

Die vorbestimmte Linie kann mit einer Seitenkante des Behälter-Trägers an der Entnahmeseite übereinstimmen, oder parallel zu dieser verlaufen. Die Entnahme-Schwenkachse kann in diesem Fall auch in Bezug auf die Seitenkante des Behälter-Trägers definiert werden, nämlich dass die Entnahme-Schwenkachse in einem Winkel von über 0° bis 45°, vorzugsweise etwa 1° bis etwa 15°, besonders bevorzugt etwa 3° bis etwa 10°, zur Seitenkante des Behälter-Trägers angeordnet ist.

Die vorbestimmte Linie kann auch mit der Erstreckungsrichtung einer Klemmschiene der Positioniervorrichtung übereinstimmen oder parallel zu dieser verlaufen. Die Entnahme-Schwenkachse kann in diesem Fall auch in Bezug auf die Erstreckungsrichtung der Klemmschiene definiert werden, nämlich dass die Entnahme-Schwenkachse in einem Winkel von über 0° bis 45°, vorzugsweise etwa 1° bis etwa 15°, besonders bevorzugt etwa 3° bis etwa 10°, zur Erstreckungsrichtung der Klemmschiene angeordnet ist.

Der Behälter-Träger kann einen Anschlag für die Entnahmeseite des Behälters aufweisen, wobei in diesem Fall die vorbestimmte Linie mit der Erstreckungsrichtung des Anschlags übereinstimmen oder parallel zu dieser verlaufen kann. Die Entnahme-Schwenkachse kann in diesem Fall auch in Bezug auf die Erstreckungsrichtung des Anschlags definiert werden, nämlich dass die Entnahme-Schwenkachse in einem Winkel von über 0° bis 45°, vorzugsweise etwa 1° bis etwa 15°, besonders bevorzugt etwa 3° bis etwa 10°, zur Erstreckungsrichtung des Anschlags angeordnet ist.

Falls der Behälter-Träger in der Draufsicht eine symmetrische Ausbildung hat, kann die vorbestimmte Linie parallel zu einer Symmetrieachse des Behälter-Trägers verlaufen. Die Entnahme-Schwenkachse kann in diesem Fall auch in Bezug auf die Symmetrieachse des Behälter-Trägers definiert werden, nämlich dass die Entnahme-Schwenkachse in einem Winkel von über 0° bis 45°, vorzugsweise etwa 1° bis etwa 15°, besonders bevorzugt etwa 3° bis etwa 10°, zur Symmetrieachse des Behälter-Trägers angeordnet ist.

Der Behälter-Träger ist schwenkbar an der Bewegungsvorrichtung gelagert und kann relativ zu der Bewegungsvorrichtung verschwenkt werden. Diese Schwenkbewegung ist unabhängig von der Mischbewegung des Behälter-Trägers und wird grundsätzlich nicht während der Mischbewegung ausgeführt. Die Schwenkbarkeit des Behälter-Trägers gegenüber der Bewegungsvorrichtung ermöglicht ein Verlagern des Behälter-Trägers von der Mischlage in eine Entnahmelage. Entsprechend ermöglicht die Schwenkbarkeit des Behälter-Trägers gegenüber der Bewegungsvorrichtung ein Verlagern des auf dem Behälter-Träger angeordneten Behälters von einer Mischstellung in eine Entnahmestellung. Das Verlagern von der Mischlage bzw. Mischstellung in die Entnahmelage bzw. Entnahmestellung kann auch als "Aufstellen" bezeichnet werden.

In der Entnahmestellung ist der Behälter derart ausgerichtet, dass die Entnahmeseite in vertikaler Richtung an der unteren Seite des Behälters befindlich ist sowie zu einem Entnahmebereich des Behälters hin geneigt ist. Der Entnahmebereich kann im Bereich einer der unteren Ecken des Behälters angeordnet sein, wobei der Entnahmebereich direkt in der Ecke selbst oder an der oberen Fläche oder Auflagefläche des Behälters nahe der Ecke angeordnet sein kann. In der Entnahmestellung ist die Auflagefläche des Behälters in einem steilen Winkel zur horizontalen Ebene geneigt, und die Entnahmeseite des Behälters bildet die untere Seite bzw. Kante des Behälters. Die Entnahmeseite des Behälters ist zu der horizontalen Ebene geneigt, jedoch in einem flacheren Winkel. Wenn die Auflagefläche in etwa senkrecht zur horizontalen Ebene aufgestellt ist, entspricht der Neigungswinkel der Entnahmeseite zur horizontalen Ebene in etwa dem Schwenkachsenwinkel zwischen der Entnahme-Schwenkachse und der vorbestimmten Linie des Behälter-Trägers. Wenn die Auflagefläche weniger als 90° zur horizontalen Ebene geneigt ist, ist der Neigungswinkel der Entnahmeseite zur horizontalen Ebene geringer als der Schwenkachsenwinkel zwischen der Entnahme-Schwenkachse und der vorbestimmten Linie des Behälter-Trägers. Die Auflagefläche des Behälters kann in der Entnahmestellung in einem Winkel von 30° bis 90°, vorzugsweise 35° bis 55°, zur horizontalen Ebene geneigt sein.

In anderen Worten ausgedrückt ist in der Entnahmelage die Aufnahmefläche des Behälter-Trägers in einem steilen Winkel zur horizontalen Ebene geneigt, und die vorbestimmte Linie des Behälter-Trägers ist ebenfalls zu der horizontalen Ebene geneigt, jedoch in einem flacheren Winkel. Wenn die Aufnahmefläche in etwa senkrecht zur horizontalen Ebene aufgestellt ist, entspricht der Neigungswinkel der vorbestimmten Linie zur horizontalen Ebene in etwa dem Schwenkachsenwinkel zwischen der Entnahme-Schwenkachse und der vorbestimmten Linie. Wenn die Aufnahmefläche weniger als 90° zur horizontalen Ebene geneigt ist, ist der Neigungswinkel der vorbestimmten Linie zur horizontalen Ebene geringer als der Schwenkachsenwinkel zwischen der Entnahme-Schwenkachse und der vorbestimmten Linie. Die Aufnahmefläche des Behälter-Trägers kann in der Entnahmelage in einem Winkel von 30° bis 90°, vorzugsweise 35° bis 55°, zur horizontalen Ebene geneigt sein.

Da in der Mischlage bzw. Ruhelage, d.h. wenn der Behälter in der Mischstellung bzw. Ruhestellung ist, der Behälter-Träger (bzw. die Aufnahmefläche davon) im Wesentlichen zur horizontalen Ebene ausgerichtet ist, gelten die obigen Ausführungen zu den Neigungen in Relation zu der horizontalen Ebene im Wesentlichen auch für die Neigungen in Relation zu der Mischlage bzw. Ruhelage.

Die Entnahme-Schwenkachse ist in einem Schwenkachsenwinkel von über 0° bis 45° zur vorbestimmten Linie des Behälter-Trägers angeordnet, wobei hier der kleinste Winkel (d.h. ein Winkel zwischen 0° und 90°) zwischen der Entnahme-Schwenkachse und der vorbestimmten Linie gemeint ist. Falls sich die Entnahme-Schwenkachse und die vorbestimmte Linie nicht im Raum schneiden, also windschief sind, ist der Schwenkachsenwinkel als der Winkel zwischen den Richtungsvektoren der Entnahme-Schwenkachse und der vorbestimmten Linie zu bestimmen. In anderen Worten ist der Schwenkachsenwinkel der größte ermittelbare Winkel zwischen 0° und 90° zwischen den projizierten Linien der Entnahme-Schwenkachse und der vorbestimmten Linie auf sämtlichen möglichen Ebenen im Raum.

Vorzugsweise entspricht der Schwenkachsenwinkel dem Winkel zwischen den auf die horizontale Ebene projizierten Linien der Entnahme-Schwenkachse und der vorbestimmten Linie, wenn der Behälter-Träger (bzw. die Aufnahmefläche davon) zur horizontalen Ebene ausgerichtet ist bzw. wenn der Behälter-Träger in der Mischlage oder Ruhelage ist. In anderen Worten ist vorzugsweise die Entnahme-Schwenkachse innerhalb der horizontalen Ebene gegenüber der vorbestimmten Linie um den Schwenkachsenwinkel geneigt, und weist keine Neigung gegenüber der horizontalen Ebene auf. Der Behälter-Träger und/oder die Bewegungsvorrichtung können ein Schwenklager aufweisen, welches den Behälter-Träger mit der Bewegungsvorrichtung verbindet, wobei die Schwenkachse des Schwenklagers der Entnahme-Schwenkachse entspricht.

Vorteilhafterweise ermöglicht die schräge Anordnung der Entnahme-Schwenkachse zur vorbestimmten Linie des Behälter-Trägers, dass der Behälter-Träger und der darauf positionierbare Behälter mittels einer einzigen Schwenkbewegung in eine vorteilhafte Entnahmelage bzw. Entnahmestellung verlagert werden kann. In der Entnahmelage ist der Behälter-Träger gegenüber der horizontalen Ebene aufgestellt. Der Behälter wird vorzugsweise in der horizontalen Position angebracht und entnommen. Es ist aber nicht auszuschließen, dass der Behälter auch in der Entnahmestellung am Behälter-Träger positioniert bzw. fixiert wird. Einfacher ist dies aber in der Ruhelage. Ferner ist in der Entnahmestellung die Entnahmeseite des Behälters zu einem Entnahmebereich hin geneigt, so dass der Entnahmebereich immer am tiefsten Punkt des Behälters angeordnet ist. Hierdurch können sich Zellen oder Metabolite, die sich aufgrund der Schwerkraft in dem Fluid oder der Suspension absetzen, auf vorteilhafte Weise in dem Entnahmebereich sammeln, was die Entnahme zusätzlich vereinfacht.

Vorzugsweise ist die Entnahme-Schwenkachse in einem Schwenkachsenwinkel von etwa 1° bis etwa 15°, besonders vorzugsweise etwa 3° bis etwa 10°, zur vorbestimmten Linie angeordnet.

Bei einem zu geringen Schwenkachsenwinkel kann in der Entnahmestellung die Neigung der Entnahmeseite zum Entnahmebereich hin für eine gute Sedimentation zu klein sein. Ein zu großer Schwenkachsenwinkel hingegen kann das Positionieren bzw. Fixieren des Behälters an dem Behälter-Träger erschweren, da der Behälter, der ein hohes Gewicht aufweisen kann, in einem stark von der horizontalen Ebene abweichenden Winkel an dem Behälter-Träger positioniert werden muss. Es hat sich herausgestellt, dass ein Schwenkachsenwinkel von etwa 1° bis etwa 15°, vorzugsweise etwa 3° bis etwa 10°, besonders vorteilhaft für das Sammeln von Produkten im Entnahmebereich und zur einfachen Positionierung des Behälters an dem Behälter-Träger ist.

Vorzugsweise ist die Bewegungsvorrichtung ausgelegt, den Behälter-Träger um eine im Wesentlichen horizontale Wippachse hin- und her zu bewegen, wobei die Wippachse im Wesentlichen parallel zur vorbestimmten Linie angeordnet ist.

Bei der Wipp-Bewegung wird der Behälter-Träger zunächst um etwa 2° bis 15° zur horizontalen Ebene geneigt, und anschließend in umgekehrter Richtung um etwa 2° bis 15° zur horizontalen Ebene geneigt. Diese Bewegung des Behälter-Trägers kann wiederholt ausgeführt werden und auf den Behälter übertragen werden, so dass eine ständige Wellenbewegung des Inhalts des Behälters erzeugt werden kann. Die Wippachse kann durch die Bewegungsvorrichtung vorgegeben sein. Die Wippachse ist vorzugsweise mittig zum Behälter-Träger bzw. dem darauf positionierten Behälter angeordnet, so dass der in vertikaler Richtung zurückgelegte Weg der Entnahmeseite bei der Kippbewegung etwa dem in vertikaler Richtung zurückgelegten Weg der der Entnahmeseite gegenüberliegenden Seite des Behälters entspricht. Die Wippachse kann von der Aufnahmefläche des Behälter-Trägers in vertikaler Richtung beabstandet sein. Die Wippachse ist im Wesentlichen parallel zur vorbestimmten Linie angeordnet, so dass die Entnahme-Schwenkachse auch in Bezug auf die Wippachse definiert werden kann, nämlich dass die Entnahme-Schwenkachse in einem Winkel von über 0° bis 45°, vorzugsweise etwa 1° bis etwa 15°, besonders bevorzugt etwa 3° bis etwa 10°, zur Wippachse angeordnet ist.

Vorzugsweise weist die Bewegungsvorrichtung ein bewegliches Kopplungselement auf, welches zum Bewegen des Behälter-Trägers mit dem Behälter-Träger verbunden ist, wobei der Behälter-Träger um die Entnahme-Schwenkachse schwenkbar an dem Kopplungselement gelagert ist, so dass der Behälter-Träger mittels einer Schwenkbewegung von einer Mischlage in eine Entnahmelage verlagert werden kann.

Das Kopplungselement kann von einem Antrieb der Bewegungsvorrichtung angetrieben bzw. bewegt werden. Das Kopplungselement kann von einer Basis der Bewegungsvorrichtung nach oben vorstehen. Der Behälter-Träger ist über das Kopplungselement mit der Bewegungsvorrichtung verbunden, so dass die Bewegung des Kopplungselements auf den Behälter-Träger und weiter auf den Behälter übertragen werden kann.

Vorzugsweise weist die Positioniervorrichtung mindestens eine Klemmschiene auf, welche sich im Wesentlichen entlang der vorbestimmten Linie oder parallel dazu erstreckt.

Mittels der Klemmschiene kann auf vorteilhafte Weise eine Seite des Behälters, insbesondere im Fall eines Folienbeutels, an dem Behälter-Träger fixiert werden. Vorzugsweise ist eine Klemmschiene an der Entnahmeseite des Behälters und eine Klemmschiene an der der Entnahmeseite gegenüberliegenden Seite des Behälters vorgesehen. Der Behälter kann an den entsprechenden Stellen Laschen oder Schienen aufweisen, die mit den Klemmschienen zusammenwirken können. In einer besonders bevorzugten Ausführungsform verfügt der Behälter in seiner Peripherie an mindestens 2 Seiten über in Laschen eingesteckte stabile Stäbe oder Stangen, die mit den Klemmschienen des Behälter-Trägers korrespondieren und von diesen eingeklemmt werden. Der Behälter wird so mithilfe der in den Klemmschienen fixierten Stäbe gehalten. Die stabilen Stäbe oder Stangen sind vorzugsweise aus Kunststoff.

Vorzugsweise weist die Positioniervorrichtung zumindest einen Befestigungshaken auf, welcher im Wesentlichen entlang der vorbestimmten Linie oder parallel dazu angeordnet ist.

Der Befestigungshaken ermöglicht ein besonders einfaches Fixieren einer Seite des Behälters an dem Behälter-Träger. Der Befestigungshaken kann eine sich in Breitenrichtung erstreckende Form aufweisen und/oder es können eine Mehrzahl von Befestigungshaken entlang der vorbestimmten Linie oder parallel dazu angeordnet sein. Vorzugsweise ist ein Befestigungshaken an der Entnahmeseite des Behälters und ein Befestigungshaken an der der Entnahmeseite gegenüberliegenden Seite des Behälters vorgesehen. Der Behälter kann Ausnehmungen, insbesondere Löcher und/oder Schlitze, aufweisen, in welche der oder die Befestigungshaken eingeführt werden können.

Vorzugsweise ist der Behälter-Träger wannenförmig ausgebildet, und eine Innenkante des wannenförmigen Behälter-Trägers erstreckt sich im Wesentlichen entlang der vorbestimmten Linie oder parallel dazu.

Die Wannenform des Behälter-Trägers ermöglicht eine besonders gute Abstützung bei Behältern in Form von Folienbeuteln, da diese im befüllten und aufgeblasenen Zustand eine Auflagefläche aufweisen können, die der Wannenform entspricht. Ferner wird durch die Wannenform ein guter Seitenhalt des Behälters ermöglicht, was während der Mischbewegung und beim Aufstellen wichtig ist. Bei Leckagen bietet eine geschlossene Wanne eine zusätzliche Sicherheit vor sich weiter verbreitenden Kontaminationen mit dem Fluid aus dem Behälter. Da der Behälter in diesem Fall an der wannenförmigen Aufnahmefläche des Behälter-Trägers anliegt, erstreckt sich die Innenkante des Behälter-Trägers entlang der vorbestimmten Linie oder parallel dazu. In einer weiteren Ausführungsform ist der wannenförmige Behälter-Träger an einer oder mehreren Ecken offen, so dass in diesen Bereichen bequem Zufuhr- und/oder Abfuhrleitungen sowie Anschlüsse für diverse Sensorikelemente geführt werden können.

Vorzugsweise weist das Kopplungselement einen Auflagebereich auf, welcher an einer Unterseite des Behälter-Trägers anliegt und diesen stützt, wenn der an dem Behälter-Träger positionierbare Behälter sich in der Mischstellung befindet.

In anderen Worten stellt der Auflagebereich des Kopplungselements einen Anschlag für den Behälter-Träger dar, wenn der Behälter-Träger von der Entnahmelage in die Mischlage verlagert wird. Durch den Auflagebereich wird der Behälter-Träger in der Mischlage im Wesentlichen horizontal ausgerichtet. Hierbei wird der Behälter-Träger einerseits durch das Schwenklager zwischen Behälter-Träger und Kopplungselement und durch den Auflagebereich des Kopplungselements gestützt.

Vorzugsweise ist das Kopplungselement im Wesentlichen plattenförmig ausgebildet, wobei die Entnahme-Schwenkachse an einem Ende des plattenförmigen Kopplungselements angeordnet ist.

Im Wesentlichen plattenförmig bedeutet, dass das Kopplungselement eine flächige Ausbildung hat. Das Kopplungselement kann jedoch Ausnehmungen aufweisen oder eine Rahmenkonstruktion sein. Vorzugsweise ist das plattenförmige Kopplungselement im Wesentlichen zur horizontalen Ebene ausgerichtet, und an einem Ende des Kopplungselements ist die Entnahme-Schwenkachse bzw. das Schwenklager angeordnet. In der Mischlage kann der Behälter-Träger mit seiner Unterseite an dem Kopplungselement, insbesondere dem Auflagebereich davon, anliegen, wobei der Behälter-Träger parallel zum Kopplungselement ausgerichtet ist.

Vorzugsweise umfasst die Mischvorrichtung einen Aufstellmechanismus, welcher es ermöglicht, den Behälter-Träger in einem vorbestimmten bzw. vorbestimmbaren Aufstellwinkel relativ zur Bewegungsvorrichtung festzustellen.

Der Aufstellmechanismus ist vorzugsweise mit dem Kopplungselement verbunden und ermöglicht, den Behälter-Träger in einem vorbestimmten bzw. vorbestimmbaren Aufstellwinkel relativ zum Kopplungselement aufzustellen und/oder festzustellen. Der Aufstellmechanismus kann hydraulisch, pneumatisch und/oder elektrisch angetrieben sein und ein Aufstellen und/oder Feststellen des Behälter-Trägers in einem vorbestimmten bzw. vorbestimmbaren Aufstellwinkel ermöglichen. Der Aufstellmechanismus ist vorzugsweise zwischen dem Kopplungselement und dem Behälter-Träger angeordnet.

Vorzugsweise weist der Aufstellmechanismus einen Hebel zur Arretierung bzw. einen Arretierhebel auf, welcher schwenkbar an der Bewegungsvorrichtung angeordnet ist, und welcher den Behälter-Träger stützt, wenn der an dem Behälter-Träger positionierbare Behälter sich in der Entnahmestellung befindet.

Der Arretierhebel ist vorzugsweise schwenkbar an dem Kopplungselement angeordnet. Der Arretierhebel ermöglicht einen besonders einfachen Aufstellmechanismus, da der Behälter-Träger, insbesondere die Unterseite davon, nicht speziell konfiguriert werden muss. In der Mischlage ist der Arretierhebel im Wesentlichen horizontal und im Wesentlichen in derselben Ebene wie der Behälter-Träger und/oder das Kopplungselement ausgerichtet. Durch Aufschwenken des Arretierhebels wird der Behälter-Träger angehoben und um die Entnahme-Schwenkachse schwenkend aufgestellt, wobei der Arretierhebel an der Unterseite des Behälter-Trägers entlang gleitet. Vorzugsweise ist die Schwenkachse des Arretierhebels in etwa parallel zur Entnahme-Schwenkachse angeordnet.

Im Folgenden wird ein Ausführungsbeispiel der vorliegenden Erfindung anhand der beigefügten Figuren näher beschrieben.

Es zeigen:
- **Fig. 1**: eine Mischvorrichtung gemäß eines Ausführungsbeispiels in einer perspektivischen Ansicht.
- **Fig. 2**: die Mischvorrichtung mit einem darauf positionierten Behälter in einer Frontansicht.
- **Fig. 3**: die Mischvorrichtung mit einem darauf positionierten Behälter in einer Frontansicht mit Klemmleisten.
- **Fig. 4**: die Mischvorrichtung in einer weiteren perspektivischen Ansicht.
- **Fig. 5**: die Mischvorrichtung in einer Draufsicht.

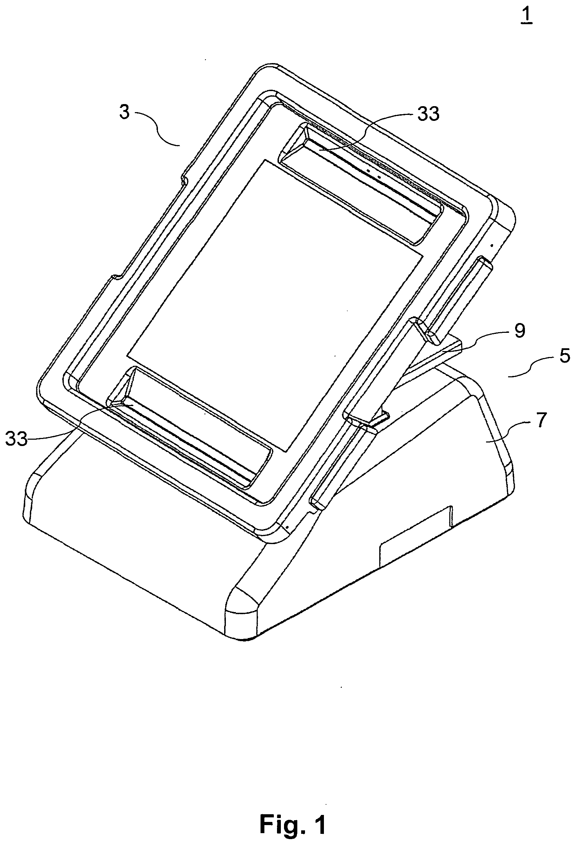

Fig. 1 zeigt eine Mischvorrichtung 1 gemäß eines Ausführungsbeispiels in einer perspektivischen Ansicht. Die Mischvorrichtung 1 umfasst einen Behälter-Träger 3, welcher auf einer Bewegungsvorrichtung 5 angeordnet ist. Die Bewegungsvorrichtung 5 umfasst eine Basis 7 und ein Kopplungselement 9. Der Behälter-Träger 3 ist mit dem Kopplungselement 9 verbunden und um eine Entnahme-Schwenkachse schwenkbar an diesem gelagert. Die Entnahme-Schwenkachse ist in Fig. 1 nicht dargestellt. In dem in Fig. 1 gezeigten Zustand ist der Behälter-Träger 3 zur horizontalen Ebene geneigt und daher nicht in der Mischlage sondern in der Entnahmelage oder einer Zwischenlage zwischen der Mischlage und der Entnahmelage. Der Behälter-Träger 3 ist plattenförmig oder wannenförmig ausgebildet und weist in seinem unteren und oberen Bereich jeweils eine Vertiefung oder Ausnehmung 33 auf. Die Ausnehmung 33 dient zur Aufnahme von Sensoren, kann jedoch auch zum Durchführen von Leitungen und/oder Kabeln Durchgänge aufweisen.

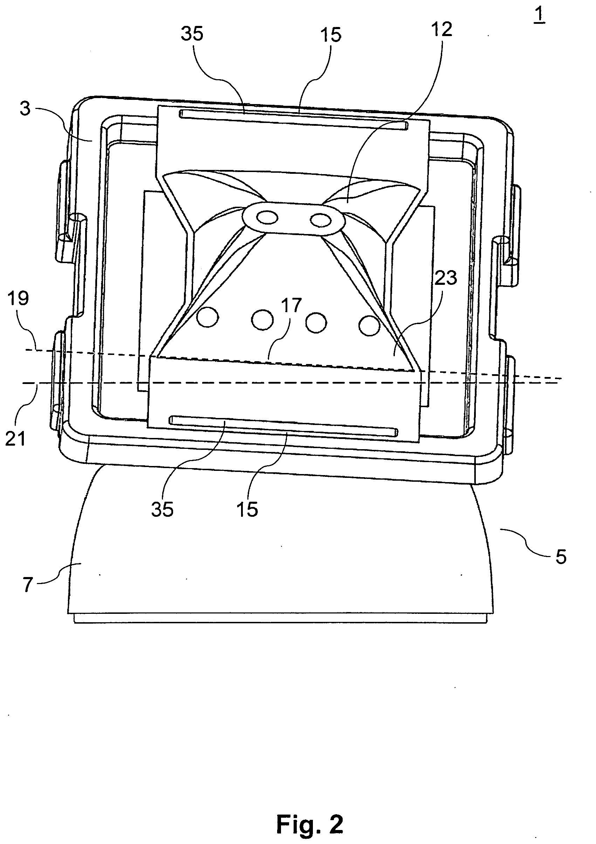

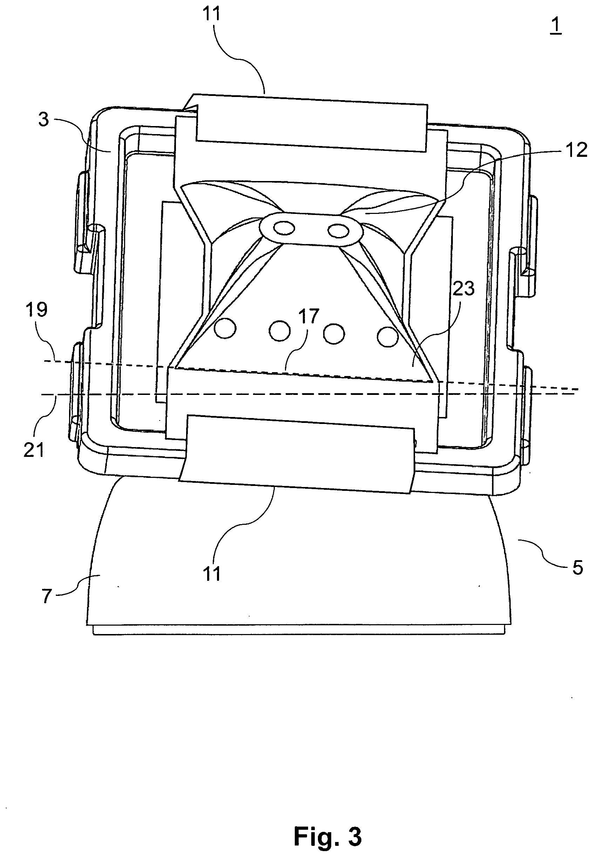

Fig. 2 und Fig. 3 zeigen jeweils die Mischvorrichtung 1 in einer Frontansicht, wobei ein Behälter 12 in Form eines Folienbeutels bzw. Bioreaktorbeutels auf dem Behälter-Träger 3 angeordnet ist. In Fig. 3 ist die Positioniervorrichtung 11 gezeigt, in Fig. 2 ist diese ausgeblendet. Der Behälter 12 ist mit zwei gegenüberliegenden Enden 15 mittels der Positioniervorrichtung 11 an dem Behälter-Träger 3 positioniert bzw. befestigt. In der gezeigten Ausführungsform wird der Behälter 12 über flexible Stäbe bzw. Klemmstäbe 35 (auch "Rods" genannt), welche in Laschen an den Enden des Behälters 12 eingelegt sind, und der Positioniervorrichtung 11 in Form von Klemmleisten befestigt. Die Klemmstäbe 35 können in dem Behälter 12 an- bzw. eingeschweißt oder aber lose in Kunststofflaschen eingeschoben sein. Durch die Positioniervorrichtung 11, insbesondere im Zusammenspiel mit den Klemmstäben 35, ist die Position des Behälters 12 auf dem Behälter-Träger 3 vorbestimmt, und die Entnahmeseite 17 des Behälters 12 ist entlang der vorbestimmten Linie 19 des Behälter-Trägers 3 angeordnet. Die Entnahme-Schwenkachse 21 ist gegenüber der vorbestimmten Linie 19 bzw. der Entnahmeseite 17 geneigt. Aus dem in Fig. 2 und Fig. 3 vorgegebenen Blickwinkel ist der genaue Schwenkachsenwinkel jedoch nicht erkennbar. Dieser wäre lediglich erkennbar, wenn der Behälter-Träger 3 senkrecht zur horizontalen Ebene aufgestellt werden würde, also senkrecht zur Blickrichtung in Fig. 2 und Fig. 3 angeordnet werden würde. Im Ausführungsbeispiel ist die Entnahme-Schwenkachse 21 horizontal ausgerichtet. Die Entnahme-Schwenkachse 21 ist durch das Schwenklager zwischen dem Behälter-Träger 3 und dem Kopplungselement 9 vorgegeben. Wenn keine Mischbewegung ausgeführt wird, ist die Entnahme-Schwenkachse 21 fest, insbesondere bewegt sich die Entnahme-Schwenkachse 21 nicht, wenn der Behälter-Träger 3 von der Mischlage in die Entnahmelage verlagert wird. Im in Fig. 2 und Fig.3 gezeigten Zustand ist der Behälter-Träger 3 in der Entnahmelage oder der Zwischenlage, d.h. der Behälter 12 ist in der Entnahmestellung oder in einer Zwischenstellung. Hierbei ist die Entnahmeseite 17 zur horizontalen Ebene geneigt, insbesondere zu dem Entnahmebereich 23 hin geneigt.

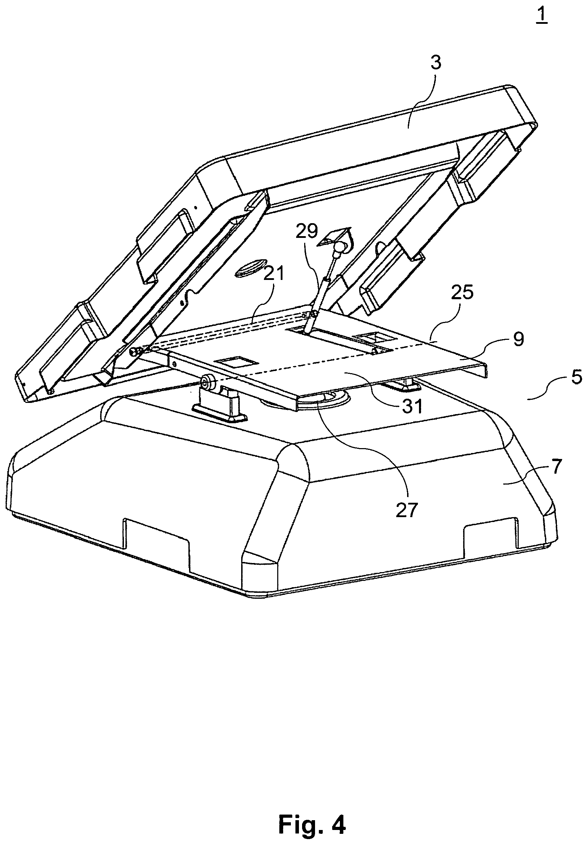

Fig. 4 zeigt die Mischvorrichtung 1 in einer weiteren perspektivischen Ansicht aus einem anderen Blickwinkel. Das Kopplungselement 9 ist plattenförmig ausgebildet und ist um die Kippachse 25 schwenkbar an der Basis 7 der Bewegungsvorrichtung 5 gelagert. Der Antrieb 27 erzeugt eine Kippbewegung des Kopplungselements 9 um die Kippachse 25 zum Erzeugen der Mischbewegung. An einem quer zur Kippachse 25 liegenden Ende des Kopplungselements 9 ist die Entnahme-Schwenkachse 21 angeordnet. Die Entnahme-Schwenkachse 21 und die Kippachse 25 liegen also in derselben horizontalen Ebene. Die Entnahme-Schwenkachse 21 ist jedoch um den Schwenkachsenwinkel zu der Kippachse 25 geneigt. Die Entnahme-Schwenkachse 21 ist auch zu der nicht gezeigten vorbestimmten Linie um den Schwenkachsenwinkel geneigt. In der Mischlage sind die vorbestimmte Linie und die Kippachse 25 parallel zueinander angeordnet. In der Mischlage liegt die Unterseite des Behälter-Trägers 3 auf dem Auflagebereich 31 des Kopplungselements 9 auf und wird durch diesen gestützt. Zum Aufstellen und/oder Feststellen des Behälter-Trägers 3 weist die Mischvorrichtung 1 einen Aufstellmechanismus 29 in Form einer Gasdruckfeder 29 auf. Die Gasdruckfeder 29 ist mit einem Ende an dem Kopplungselement 9 und mit dem anderen Ende an der Unterseite des Behälter-Trägers 3 befestigt.

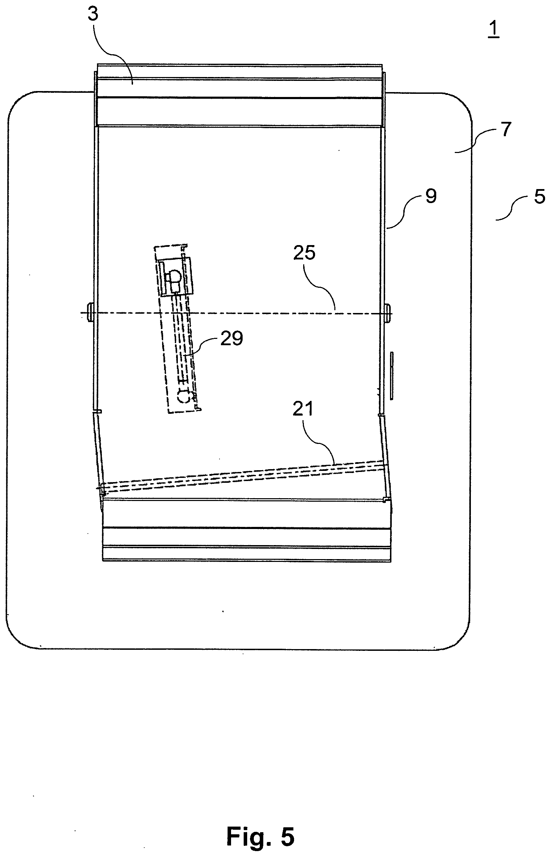

Fig. 5 zeigt die Mischvorrichtung 1 in einer Draufsicht. Die Entnahme-Schwenkachse 21 ist um den Schwenkachsenwinkel geneigt zur Kippachse 25 angeordnet. Die Gasdruckfeder 29 ist derart ausgerichtet, dass durch lineares Verfahren der Gasdruckfeder 29 der Behälter-Träger 3 von der Mischlage in die Entnahmelage verlagert werden kann. Hierzu ist die Gasdruckfeder 29 in der Ausgangslage, d.h. wenn der Behälter-Träger 3 in der Mischlage ist und die Gasdruckfeder 29 parallel zum Kopplungselement 9 in der horizontalen Ebene angeordnet ist, in etwa um dem Schwenkachsenwinkel zu einer Linie geneigt, welche senkrecht zu der Kippachse 25 und/oder der vorbestimmten Linie steht. In anderen Worten ist die Gasdruckfeder-Schwenkachse in etwa parallel zur Entnahme-Schwenkachse 21 angeordnet.

### Bezugszeichenliste

- 1: Mischvorrichtung
- 3: Behälter-Träger
- 5: Bewegungsvorrichtung
- 7: Basis der Bewegungsvorrichtung
- 9: Kopplungselement
- 11: Positioniervorrichtung
- 12: Behälter
- 15: Behälterende
- 17: Entnahmeseite
- 19: vorbestimmte Linie
- 21: Entnahme-Schwenkachse
- 23: Entnahmebereich
- 25: Kippachse
- 27: Antrieb
- 29: Aufstellmechanismus, Gasdruckfeder
- 31: Auflagebereich
- 33: Ausnehmung
- 35: Klemmstab

## Patentansprüche

1. Mischvorrichtung (1) zum Mischen eines Inhalts eines Behälters (12), aufweisend:
einen Behälter-Träger (3) zum Tragen des Behälters (12); und
eine Bewegungsvorrichtung (5), welche zum Bewegen des Behälter-Trägers (3) mit dem Behälter-Träger (3) verbunden ist, um den Inhalt des Behälters (12) in einer Mischstellung mischen zu können;
wobei der Behälter-Träger (3) eine Positioniervorrichtung (11) aufweist, welche ausgelegt ist, den Behälter (12) in einer vorbestimmten bzw. vorbestimmbaren Lage an dem Behälter-Träger (3) zu positionieren, so dass eine Entnahmeseite (17) des Behälters (12) im Wesentlichen entlang einer vorbestimmten Linie (19) des Behälter-Trägers (3) angeordnet ist,
wobei der Behälter-Träger (3) um eine Entnahme-Schwenkachse (21) schwenkbar relativ zu der Bewegungsvorrichtung (5) gelagert ist, um den daran positionierbaren Behälter (12) in eine Entnahmestellung verlagern zu können,
**dadurch gekennzeichnet, dass** die Entnahme-Schwenkachse (21) derart in einem Schwenkachsenwinkel von über 0° bis 45° zur vorbestimmten Linie (19) des Behälter-Trägers (3) angeordnet ist, dass der daran positionierbare Behälter (12) von der Mischstellung mittels einer Schwenkbewegung des Behälter-Trägers (3) um die Entnahme-Schwenkachse (21) in die Entnahmestellung verlagerbar ist, in der die Entnahmeseite (17) an der unteren Seite des Behälters (12) befindlich sowie zu einem Entnahmebereich (23) des Behälters (12) hin geneigt ist.

2. Mischvorrichtung (1) nach Anspruch 1, wobei die Entnahme-Schwenkachse (21) in einem Schwenkachsenwinkel von etwa 1° bis etwa 15°, vorzugsweise etwa 3° bis etwa 10°, zur vorbestimmten Linie (19) angeordnet ist.

3. Mischvorrichtung (1) nach Anspruch 1 oder 2, wobei die Bewegungsvorrichtung (5) ausgelegt ist, den Behälter-Träger (3) um eine im Wesentlichen horizontale Wippachse (25) hin- und her zu bewegen, wobei die Wippachse (25) im Wesentlichen parallel zur vorbestimmten Linie (19) angeordnet ist.

4. Mischvorrichtung (1) nach einem der vorangehenden Ansprüche, wobei die Bewegungsvorrichtung (5) ein bewegliches Kopplungselement (9) aufweist, welches zum Bewegen des Behälter-Trägers (3) mit dem Behälter-Träger (3) verbunden ist, wobei der Behälter-Träger (3) um die Entnahme-Schwenkachse (21) schwenkbar an dem Kopplungselement (9) gelagert ist, so dass der Behälter-Träger (3) mittels einer Schwenkbewegung von einer Mischlage in eine Entnahmelage verlagert werden kann.

5. Mischvorrichtung (1) nach einem der vorangehenden Ansprüche, wobei die Positioniervorrichtung (11) zumindest eine Klemmschiene aufweist, welche sich im Wesentlichen entlang der vorbestimmten Linie (19) oder parallel dazu erstreckt.

6. Mischvorrichtung (1) nach einem der vorangehenden Ansprüche, wobei die Positioniervorrichtung (11) zumindest einen Befestigungshaken aufweist, welcher im Wesentlichen entlang der vorbestimmten Linie (19) oder parallel dazu angeordnet ist.

7. Mischvorrichtung (1) nach einem der vorangehenden Ansprüche, wobei der Behälter-Träger (3) wannenförmig ausgebildet ist, und eine Innenkante des wannenförmigen Behälter-Trägers (3) sich im Wesentlichen entlang der vorbestimmten Linie (19) oder parallel dazu erstreckt.

8. Mischvorrichtung nach einem der vorangehenden Ansprüche 4 bis 7, wobei das Kopplungselement (9) einen Auflagebereich (31) aufweist, welcher an einer Unterseite des Behälter-Trägers (3) anliegt und diesen stützt, wenn der an dem Behälter-Träger (3) positionierbare Behälter sich in der Mischstellung befindet.

9. Mischvorrichtung (1) nach einem der vorangehenden Ansprüche 4 bis 8, wobei das Kopplungselement (9) im Wesentlichen plattenförmig ausgebildet ist, wobei die Entnahme-Schwenkachse (21) an einem Ende des plattenförmigen Kopplungselements (9) angeordnet ist.

10. Mischvorrichtung (1) nach einem der vorangehenden Ansprüche, weiter umfassend einen Aufstellmechanismus (29), welcher es ermöglicht, den Behälter-Träger (3) in einem vorbestimmten bzw. vorbestimmbaren Aufstellwinkel relativ zur Bewegungsvorrichtung (5) festzustellen.

11. Mischvorrichtung (1) nach Anspruch 10, wobei der Aufstellmechanismus (29) einen Arretierhebel aufweist, welcher schwenkbar an der Bewegungsvorrichtung (5) angeordnet ist, und welcher den Behälter-Träger (3) stützt, wenn der an dem Behälter-Träger (3) positionierbare Behälter (12) sich in der Entnahmestellung befindet.

## Claims

1. A mixing device (1) for mixing a content of a container (12), comprising:
a container support (3) for supporting the container (12); and
a moving device (5) connected to the container support (3) to move the container support (3) in order to be able to mix the content of the container (12) in a mixing position;
wherein the container support (3) has a positioning device (11), which is adapted to position the container (12) in a predetermined or predeterminable position on the container support (3) so that a removal side (17) of the container (12) is arranged substantially along a predetermined line (19) of the container support (3),
wherein the container support (3) is supported pivotally about a removal pivot axis (21) relative to the moving device (5) in order to be able to displace the container (12), which can be positioned thereon, into a removal position,
**characterized in that** the removal pivot axis (21) is arranged at a pivot axis angle of about 0° to 45° to the predetermined line (19) of the container support (3) such that the container (12) positionable thereon is displaceable from the mixing position, by means of a pivoting movement of the container support (3) about the removal pivot axis (21), into the removal position, in which the removal side (17) is located on the lower side of the container (12) and is inclined toward a removal area (23) of the container (12).

2. The mixing device (1) according to claim 1, wherein the removal pivot axis (21) is arranged at a pivot axis angle of about 1° to about 15°, preferably about 3° to about 10° to the predetermined line (19).

3. The mixing device (1) according to claim 1 or 2, wherein the moving device (5) is adapted to reciprocate the container support (3) about a substantially horizontal rocking axis (25), wherein the rocking axis (25) is arranged substantially parallel to the predetermined line (19).

4. The mixing device (1) according to one of the preceding claims, wherein the moving device (5) has a movable coupling element (9) that is connected to the container support (3) for moving the container support (3), wherein the container support (3) is supported on the coupling element (9) pivotally about the removal pivot axis (21), so that the container support (3) can be displaced from a mixing position into a removal position by means of a pivoting movement.

5. The mixing device (1) according to one of the preceding claims, wherein the positioning device (11) has at least one clamping rail, which extends substantially along the predetermined line (19) or parallel thereto.

6. The mixing device (1) according to one of the preceding claims, wherein the positioning device (11) has at least one fastening hook, which is arranged substantially along the predetermined line (19) or parallel thereto.

7. The mixing device (1) according to one of the preceding claims, wherein the container support (3) is trough-shaped, and an inner edge of the wan-trough-shaped container support (3) extends substantially along the predetermined line (19) or parallel thereto.

8. The mixing device according to one of the preceding claims 4 to 7, wherein the coupling element (9) has a bearing area (31) that rests against an underside of the container support (3) and supports it when the container positionable on the container support (3) is in the mixing position.

9. The mixing device (1) according to one of the preceding claims 4 to 8, wherein the coupling element (9) is formed to be substantially plate-shaped, wherein the removal pivot axis (21) is arranged at one end of the plate-shaped coupling element (9).

10. The mixing device (1) according to one of the preceding claims, further comprising a set-up mechanism (29), which makes it possible to fix the container support (3) in a predetermined or predeterminable setting angle relative to the moving device (5).

11. The mixing device (1) according to claim 10, wherein the set-up mechanism (29) comprises a locking lever, which is pivotally arranged on the moving device (5), and which supports the container support (3), when the container (12) positionable on the container support (3) is in the removal position.

## Revendications

1. Dispositif mélangeur (1) pour mélanger le contenu d'un récipient (12), présentant :
un support de récipient (3) pour supporter le récipient (12) ; et
un dispositif de déplacement (5) relié au support de récipient (3) pour déplacer le support de récipient (3) afin de pouvoir mélanger le contenu du récipient (12) dans une position de mélangeage ;
sachant que le support de récipient (3) présente un dispositif de positionnement (11) qui est conçu pour positionner le récipient (12) dans une position prédéterminée ou prédéterminable sur le support de récipient (3) de manière qu'un côté de prélèvement (17) du récipient (12) soit disposé essentiellement le long d'une ligne prédéterminée (19) du support de récipient (3),
sachant que le support de récipient (3) est monté de manière pivotante autour d'un axe de pivotement de prélèvement (21) par rapport au dispositif de déplacement (5) pour pouvoir déplacer le récipient (12) positionnable sur celui-ci dans une position de prélèvement,
**caractérisé en ce que** l'axe de pivotement de prélèvement (21) est disposé d'après un angle d'axe de pivotement de plus de 0° à 45° par rapport à la ligne prédéterminée (19) du support de récipient (3) de manière que le récipient (12) positionnable sur celui-ci peut être déplacé de la position de mélange au moyen d'un mouvement de pivotement du support de récipient (3) autour de l'axe de pivotement de prélèvement (21) dans la position de prélèvement, dans laquelle le côté de prélèvement (17) est situé sur le côté inférieur du récipient (12) ainsi que incliné vers une zone de prélèvement (23) du récipient (12)

2. Dispositif mélangeur (1) d'après la revendication 1, sachant que l'axe de pivotement de prélèvement (21) est disposé d'après un angle d'axe de pivotement d'environ 1° à environ 15°, de préférence d'environ 3° à environ 10°, par rapport à la ligne prédéterminée (19).

3. Dispositif mélangeur (1) d'après la revendication 1 ou 2, sachant que le dispositif mobile (5) est conçu pour déplacer le support de récipient (3) en va-et-vient autour d'un axe de basculement (25) essentiellement horizontal, l'axe de basculement (25) étant disposé essentiellement de manière parallèle à la ligne prédéterminée (19).

4. Dispositif mélangeur (1) d'après l'une des revendications précédentes, sachant que le dispositif de déplacement (5) présente un élément d'accouplement mobile (9) qui est raccordé au support de récipient (3) pour déplacer le support de récipient (3), sachant que le support de récipient (3) est monté de manière pivotante autour de l'axe de pivotement de prélèvement (21) sur l'élément d'accouplement (9) de manière que le support de récipient (3) puisse être déplacé d'une position de mélangeage à une position de prélèvement au moyen d'un mouvement de pivotement.

5. Dispositif mélangeur (1) d'après l'une quelconque des revendications précédentes, sachant que le dispositif de positionnement (11) présente au moins un rail de serrage s'étendant essentiellement le long ou parallèlement à la ligne prédéterminée (19).

6. Dispositif mélangeur (1) d'après l'une des revendications précédentes, sachant que le dispositif de positionnement (11) présente au moins un crochet de fixation disposé essentiellement le long de la ligne prédéterminée (19) ou parallèlement à celle-ci.

7. Dispositif mélangeur (1) d'après l'une quelconque des revendications précédentes, sachant que le support de récipient (3) est réalisé en forme de cuve et qu'un bord intérieur du support de récipient (3) en forme de cuve s'étend essentiellement le long ou parallèlement à la ligne prédéterminée (19).

8. Dispositif mélangeur d'après l'une quelconque des revendications précédentes de 4 à 7, sachant que l'élément d'accouplement (9) présente une zone d'appui (31) qui repose contre et supporte un côté inférieur du support de récipient (3) lorsque le récipient pouvant être positionné sur le support de récipient (3) est dans la position de mélangeage.

9. Dispositif mélangeur (1) d'après l'une quelconque des revendications précédentes de 4 à 8, sachant que l'élément d'accouplement (9) est réalisé essentiellement en forme de plaque, sachant que l'axe de pivotement de prélèvement (21) est disposé à une extrémité de l'élément d'accouplement (9) en forme de plaque.

10. Dispositif mélangeur (1) d'après l'une quelconque des revendications précédentes, comprenant en outre un mécanisme d'élévation (29) qui permet de fixer le support de récipient (3) à un angle d'élévation prédéterminé ou prédéterminable par rapport au dispositif de déplacement (5).

11. Dispositif mélangeur (1) d'après la revendication 10, sachant que le mécanisme d'élévation (29) présente un levier de verrouillage disposé de manière pivotante sur le dispositif de déplacement (5) et qui soutient le support de récipient (3) lorsque le récipient (12) pouvant être positionné sur le support de récipient (3) est dans la position de prélèvement.
